(19) Europäisches Patentamt

European Patent Office

Office européen des brevets

(11) **EP 0 541 443 B1**

# (12) FASCICULE DE BREVET EUROPEEN

(45) Date de publication et mention
de la délivrance du brevet:
**31.01.1996 Bulletin 1996/05**

(51) Int. Cl.⁶: **A61F 2/06**

(21) Numéro de dépôt: **92402991.1**

(22) Date de dépôt: **05.11.1992**

(54) **Endoprothèse pour implantation transluminale**

Endoprothese zur transluminalen Implantation

Endoprosthesis for transluminal implantation

(84) Etats contractants désignés:
**AT BE CH DE DK ES FR GB GR IE IT LI LU MC NL PT SE**

(30) Priorité: **08.11.1991 FR 9113820**

(43) Date de publication de la demande:
**12.05.1993 Bulletin 1993/19**

(73) Titulaire: **Meadox Medicals, Inc.**
**Oakland New Jersey 07436 (US)**

(72) Inventeurs:
• **Ansel, Denis**
**35235 Thorigne-Fouillard (FR)**
• **Cardon, Alain**
**F-35700 Rennes (FR)**

• **Kerdiles, Yvon**
**F-35760 Saint Grégoire (FR)**
• **Boliveau, Michel**
**F-35510 Cesson Seuigne (FR)**
• **Debuigne, Jean**
**F-35135 Chantepie (FR)**

(74) Mandataire: **Le Roux, Martine et al**
**F-75340 Paris Cédex 07 (FR)**

(56) Documents cités:
**EP-A- 0 183 372         EP-A- 0 274 846**
**EP-A- 0 335 341         EP-A- 0 421 729**
**WO-A-87/00442          US-A- 4 740 207**
**US-A- 5 064 435**

**Description**

La présente invention a pour objet de nouvelles endoprothèses pour implantation transluminale et notamment de nouvelles endoprothèses vasculaires.

Des endoprothèses pour implantation transluminale (encore appelées "greffon intraluminal dilatable" ou "stent") ont été développées ces dernières années. Il s'agit de tutelles métalliques, que l'on insère à l'intérieur d'une partie du corps humain (telles les voies biliaires, les voies urinaires, le tube digestif...) et notamment par voie percutanée, à l'intérieur des vaisseaux sanguins, généralement des artères (on parle alors d'endoprothèses vasculaires). Ces tutelles sont susceptibles de se dilater radialement. On les introduit alors qu'elles présentent un premier diamètre ($d_1$) et on les implante, à l'endroit souhaité, par exemple à l'intérieur du vaisseau, en les dilatant in situ, jusqu'à ce qu'elles présentent un second diamètre ($d_2$:$d_2$>$d_1$). On peut faire intervenir pour leur dilatation radiale un ballonnnet d'angioplastie, associé à un cathéter.

On connaît plusieurs types d'endoprothèses et principalement des endoprothèses dites "souples", telles que celles décrites dans les demandes de brevet FR-A-2 525 896 et EP-A-0 183 372 et des endoprothèses dites "rigides", telles que celles décrites dans les brevets US-A-4 739 762 et 4 776 337 ou dans les demandes de brevet EP-A-0 221 570 et 0 364 787.

On propose, selon la présente invention, un nouveau type d'endoprothèses, que l'on pourrait qualifier de "mixte" puisqu'il associe des parties souples et des parties rigides. L'association, selon l'invention, de parties souples et de parties rigides dans la structure d'une endoprothèse, n'est pas quelconque.

L'endoprothèse pour implantation transluminale, objet de la présente invention, consiste en la juxtaposition alternée de parties cylindriques axialement rigides et d'au moins une partie cylindrique axialement souple ; ladite endoprothèse comportant des parties cylindriques axialement rigides en ses deux extrémités ; lesdites parties cylindriques axialement rigides étant susceptibles de s'expanser radialement de façon plastique, tandis que la(les)dite(s) partie(s) cylindrique(s) axialement souple(s) est(sont) susceptible(s) de s'expanser radialement de façon élastique.

La juxtaposition alternée des parties souples et des parties rigides est telle que l'on trouve toujours :

- une partie axialement rigide à chaque extrémité de l'endoprothèse ;
- eux parties axialement rigides de part et d'autre d'une partie axialement souple.

Un tel agencement permet aux endoprothèses de l'invention un bon accrochage aux parois des parties du corps humain dans lesquelles elles sont implantées et minimise les perturbations du flux des fluides qui circulent dans lesdites parties du corps humain.

Les endoprothèses de l'invention peuvent exister selon de nombreuses variantes. En effet, le nombre d'alternances parties rigides/partie(s) souple(s) peut être variable.

On peut trouver une unique partie souple entre deux parties rigides d'extrémité ou plusieurs parties souples et au moins une partie rigide intermédiaire entre deux parties rigides d'extrémité.

Selon deux variantes préférées de l'invention, les structures des endoprothèses répondent aux séquences ci-après :

1) partie rigide d'extrémité - partie souple - partie rigide d'extrémité

$$R - S - R$$

2) partie rigide d'extrémité - partie souple - partie rigide intermédiaire - partie souple - partie rigide d'extrémité

$$R - S - R' - S - R$$

Pour des raisons de symétrie de déformation et d'automatisation de fabrication, il est préférable que les parties rigides intermédiaires soient identiques aux parties rigides d'extrémité (R=R'). Ceci n'est toutefois pas une obligation.

D'une manière générale et pour les raisons invoquées ci-dessus, les endoprothèses de l'invention présentent avantageusement des parties rigides (d'extrémité et en intermédiaire(s)) identiques entre elles ainsi que des parties souples identiques entre elles. On ne saurait toutefois exclure du cadre de l'invention des endoprothèses de structure plus dissymétrique.

Selon la longueur totale souhaitée et la souplesse axiale requise pour une endoprothèse de l'invention, on peut jouer :

- sur les caractéristiques intrinsèques desdites parties rigides et souples ; et notamment leur longueur,
- sur le nombre d'alternances (partie rigide/partie souple).

On propose donc selon l'invention des endoprothèses "articulées", de longueur et de souplesse axiale modulables.

Les parties souple(s) et rigides desdites endoprothèses sont solidarisées par soudure.

Elles doivent donc être en des matériaux soudables entre eux. Par ailleurs, il est évident que lesdits matériaux doivent être biocompatibles et présenter chacun les propriétés mécaniques requises (élasticité pour la partie souple, plasticité pour la partie rigide).

On préconise pour les parties rigides l'utilisation d'acier inoxydable (notamment de type 316 L) ou de titane (notamment de type T 35 ou T 40). Ces matériaux présentent, à l'état recuit, un domaine de plasticité équivalent et suffisamment important. L'utilisation de tout autre métal (ou alliage métallique) biocompatible et possédant des caractéristiques mécaniques voisines peut être envisagée.

On associe avantageusement à des parties rigides, en titane, une ou plusieurs partie(s) souple(s) en titane, présentant de bonnes propriétés élastiques, tel celui de type T 60. De la même façon, on associe avantageusement à des parties rigides, en acier inoxydable, une ou plusieurs partie(s) souple(s) en un acier inoxydable présentant l'élasticité requise, acier ayant éventuellement subi les traitements thermiques et/ou mécaniques appropriés.

En tout état de cause, les parties axialement souples susceptibles de s'expanser radialement de façon élastique sont des parties qui s'expansent radialement en conservant le matériau qui les constitue dans son domaine de déformation élastique.

Les endoprothèses de l'invention présentent toujours, comme précisé ci-dessus, des extrémités rigides. Elles peuvent par ailleurs inclure dans leur structure des parties rigides intermédiaires. Ces parties rigides - intermédiaire(s) ou d'extrémité - facilitent la mise en place de l'endoprothèse et, en tout état de cause, une fois expansées, assurent notamment son ancrage.

On optimise avantageusement ledit ancrage en donnant auxdites parties rigides des formes telles que leurs extrémités "rebiquent" lors de l'expansion radiale. Lesdites extrémités, en se déformant ainsi - ouverture vers l'extérieur -, s'incrustent légèrement dans la paroi des parties du corps où l'endoprothèse est implantée, d'où le meilleur ancrage. Ce meilleur ancrage permet de minimiser les turbulences, inhérentes à la présence de l'endoprothèse ; dans le flux sanguin par exemple si l'endoprothèse est implantée dans un vaisseau sanguin.

On peut imaginer, pour lesdites parties axialement rigides des endoprothèses de l'invention, plusieurs formes ; des formes qui, réalisées dans le matériau adéquat, permettent l'expansion radiale souhaitée et favorisent le "rebicage" évoqué ci-dessus.

Ainsi, lesdites parties cylindriques axialement rigides peuvent consister en des tubes, selon les génératrices desquels sont distribuées de façon régulière des lumières et des "demi-lumières" ; lesdites "demi-lumières" fendues, débouchant sur les extrémités desdits tubes et générant ainsi des ailettes, sur lesquelles sont avantageusement solidarisées les parties cylindriques axialement souples.

Lesdites ailettes - extrémités en T - se déforment vers l'extérieur au cours de l'expansion et sont avantageusement utilisées pour la solidarisation des parties souple(s) et rigides. Elles assurent un impactage optimum de l'endoprothèse dans la paroi des parties du corps où elles sont implantées de par leur forme et leur déformation. Elles offrent une surface d'appui certaine et tendent à s'incruster dans ladite paroi.

Les parties cylindriques axialement rigides des endoprothèses de l'invention peuvent donc consister en des tubes selon les génératrices desquels on a découpé des lumières. Le diamètre desdits tubes est évidemment celui souhaité pour l'endoprothèse, avant expansion radiale.

Les lumières découpées dans lesdits tubes sont notamment destinées à minimiser le taux de recouvrement de la paroi des parties du corps dans lesquels ils sont implantés et à permettre l'expansion radiale.

Selon une première variante, on peut prévoir une alternance d'une lumière complète - selon pratiquement toute la génératrice du tube - et d'un ensemble de deux demi-lumières, séparées par une bande métallique ; lesdites deux demi-lumières étant fendues pour déboucher sur les extrémités du tube et générer des ailettes.

Selon une seconde variante, et notamment pour obtenir des parties rigides plus longues, on peut prévoir une alternance :

- de deux lumières complètes selon une génératrice et,
- d'un ensemble de deux "demi-lumières" séparées par une lumière complète selon une autre génératrice ; les parties extrêmes desdites demi-lumières étant toujours fendues pour permettre l'expansion et former les ailettes.

On peut également prévoir, pour obtenir des parties rigides plus longues, la première variante ci-dessus, avec des lumières plus longues.

Lesdites lumières et "demi-lumières" sont distribuées sur le pourtour du tube de façon régulière ; ce, afin d'obtenir au cours de l'expansion radiale une déformation homogène.

Pour une parfaite homogénéité de ladite déformation, on optimisera avantageusement les dimensions et la distribution des découpes du tube, de sorte que toutes les parties métalliques - restantes - dudit tube aient sensiblement la même largeur ( $e \approx e' \approx e''$ ).

Ces parties métalliques pleines dudit tube consistent :

- en les bandes longitudinales entre deux séries de découpes successives selon deux génératrices ;
- en les espaces pleins entre deux découpes selon une même génératrice ;
- en les espaces pleins entre l'extrémité du tube et les extrémités, fendues ou non, desdites découpes.

Par ailleurs, selon une variante préférée de l'invention, toutes les découpes du tube ont leurs extrémités arrondies, ne présentent pas d'angle vif. On évite ainsi les risques d'amorces de fissuration, susceptibles de conduire à la rupture lors de l'expansion.

Lesdites découpes se présentent avantageusement sous la forme de rectangles, auxquels on a accolé, suivant leurs deux largeurs, des demi-cercles, de diamètre égal auxdites largeurs

De telles découpes se déforment à l'expansion pour générer des "losanges", tronqués selon leur plus grande diagonale.

Les parties cylindriques axialement rigides des endoprothèses de l'invention sont réalisées à partir de tubes métalliques sur lesquels on a effectué les découpes adéquates.

Leur taux de recouvrement, après expansion, doit être inférieur à 20 %. Ce chiffre est un compromis. Il convient évidemment qu'il y ait suffisamment de matériau pour que l'endoprothèse joue son rôle de tutelle mais qu'il n'y en ait pas trop pour que ladite endoprothèse ne gêne pas ; ne constitue pas par exemple un appel à thrombose à l'intérieur d'un vaisseau sanguin.

En ce qui concerne les parties cylindriques axialement souples, on a vu qu'elles étaient toujours insérées, dans la structure des endoprothèses de l'invention, entre deux parties cylindriques axialement rigides. Ce sont elles qui confèrent auxdites endoprothèses leur souplesse axiale.

Elles consistent avantageusement en un maillage de fils, chacun desdits fils étant en ses deux extrémités, soudé aux parties cylindriques axialement rigides - qui entourent lesdites parties cylindriques axialement souples - à proximité d'un noeud dudit maillage.

Toutes les extrémités de fils de la partie maillée souple sont solidarisées aux parties rigides. Ceci garantit le "non-détricotage" de la partie maillée et prévient contre l'existence de tout fil libre, risquant soit de provoquer une blessure de la paroi des parties du corps humain dans lesquelles l'endoprothèse est implantée, soit au contraire de flotter à l'intérieur desdites parties et de générer ainsi des turbulences néfastes.

Les extrémités des parties souples - soutenues en expansion par les parties rigides - sont ainsi stabilisées.

La solidarisation par soudure est réalisée à proximité d'un point de concours des fils du maillage ; ce, dans le but de minimiser les efforts, au niveau de ladite soudure, au cours de l'expansion.

Il en résulte la possibilité d'ajuster la longueur des parties souples à une demi-maille près. La longueur d'une partie souple entre deux parties rigides est évidemment comprise entre une longueur minimale, nécessaire à l'obtention de l'élasticité requise et une longueur maximale au-delà de laquelle un risque d'affaissement de la zone médiane de ladite partie souple est susceptible d'apparaître.

Le maillage des fils qui constituent les parties souples des endoprothèses de l'invention n'est avantageusement pas quelconque.

Les différentes parties - souple(s) et rigide(s) - desdites endoprothèses, susceptibles de s'expanser radialement, sont en fait dilatées in situ - à l'intérieur d'un vaisseau sanguin ou de toute autre partie du corps humain - sous l'action d'un ballonnet d'expansion. Un tel ballonnet est à base d'une structure tissée. On peut notamment en utiliser un en polyuréthanne associé à un cathéter de dilatation.

On fera alors avantageusement coïncider les caractéristiques des mailles constituant les parties souples des endoprothèses de l'invention avec celles de la structure tissée dudit ballonnet d'expansion. Par caractéristiques des mailles, on entend :

- l'angle au repos (avant expansion)
- l'angle ouvert (après expansion) ; angles mesurés par rapport à l'axe longitudinal de l'ensemble ;
- le comportement à l'expansion.

Une telle coïncidence assure un déplacement axial synchrone entre le ballonnet et l'endoprothèse, lors de l'expansion, annihilant ainsi tout glissement relatif de l'un par rapport à l'autre. Elle garantit ainsi l'absence de toute déformation axiale néfaste lors de la mise en place de l'endoprothèse.

Les parties cylindriques axialement souples des endoprothèses de l'invention consistent donc avantageusement en un maillage de fils métalliques, évoluant en hélice d'une partie cylindrique axialement rigide à la partie cylindrique axialement rigide suivante.

Le taux de recouvrement des parties cylindriques axialement souples est généralement voisin de celui des parties cylindriques axialement rigides ; il est inférieur à 20 %, de préférence voisin de 15 %.

Les soudures des fils des parties souples aux parties rigides sont comme indiqué ci-dessus réalisées de façon à minimiser, à leur niveau, les contraintes et la surépaisseur. Lesdites soudures peuvent avantageusement être localisées sur la zone de moindre déformation des ailettes, constituant les extrémités des parties rigides décrites ci-dessus. Elles sont effectuées sur les parties externes desdites ailettes.

Selon une variante préférée de l'invention, l'endoprothèse comporte :

- des parties cylindriques axialement rigides, qui consistent en des tubes selon les génératrices desquels sont distribuées de façon régulière des lumières et des "demi-lumières", lesdites "demi-lumières" fendues débouchant sur les extrémités desdits tubes et générant ainsi des ailettes ;
- au moins une partie cylindrique axialement souple, qui consiste en un maillage de fils ; les fils dudit maillage étant, à proximité d'un noeud de celui-ci, soudés par paire, un à chaque extrémité desdites ailettes ; un en hélice pas à droite, l'autre en hélice pas à gauche.

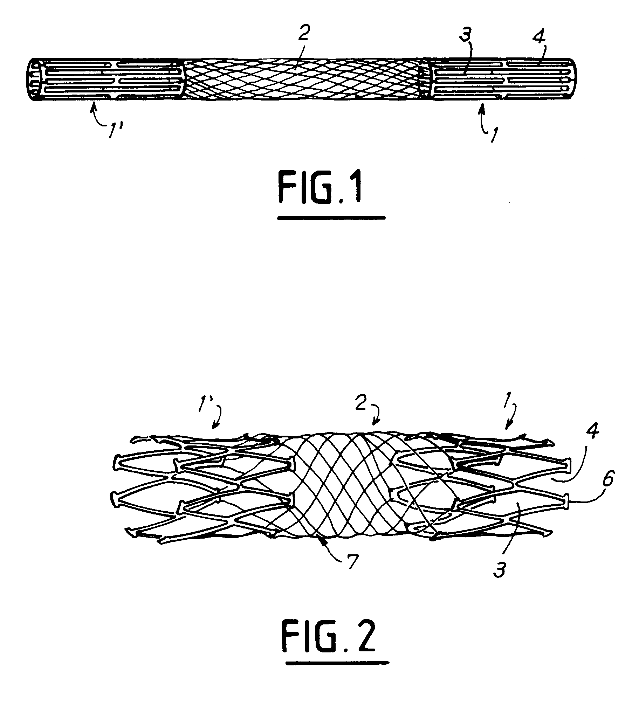

L'invention va maintenant être décrite en référence aux figures 1 à 4 annexées.

La figure 1 est une vue en perspective d'une endoprothèse selon l'invention, non expansée.

La figure 2 est une vue en perspective de cette même endoprothèse, expansée.

La figure 3 est une vue partielle de face d'une zone de soudure partie souple/partie rigide de l'endoprothèse selon la figure 1 (à l'état non expansé).

La figure 4 représente cette même zone, à l'état expansé.

Sur lesdites figures, on a représenté une endoprothèse comportant une partie cylindrique axialement souple (2) entre deux parties cylindriques axialement rigides (1, 1'). Lesdites parties rigides (1, 1') comportent des lumières (3) et des "demi-lumières" (4), fendues en (5).

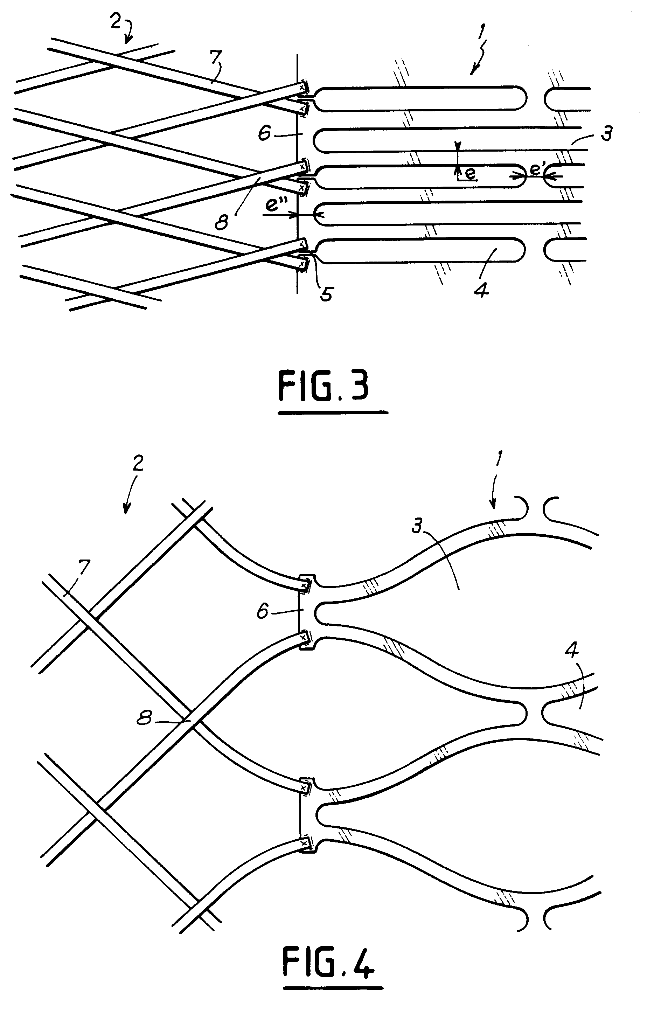

On a représenté en (6) les ailettes générées par de telles découpes (3 et 4). Toutes ces découpes ont leurs extrémités arrondies. On notera plus particulièrement sur la figure 3 que les parties métalliques pleines des tubes, constituant les extrémités rigides de l'endoprothèse, ont toutes sensiblement la même largeur :

$$e \approx e' \approx e''$$

La partie cylindrique axialement souple (2) est un maillage de fils (7) de section cylindrique. Lesdits fils s'entrecroisent en des noeuds (8). Ils sont soudés à proximité desdits noeuds (8), par paire, sur les extrémités des ailettes (6) des parties rigides (1, 1').

A titre d'exemple, on précise ci-après les dimensions que peut présenter une telle endoprothèse :

- longueur des parties rigides d'extrémité : environ 8 mm
- longueur de la partie souple médiane : de 7 à 21 mm
- diamètre initial : 3 mm
- diamètre après expansion : 9 mm.

Lesdites parties rigides d'extrémité comportent 8 lumières complétes sur leur circonférence (8 "losanges" après dilatation).

Les parties métalliques pleines - restant après les découpes - desdites parties rigides d'extrémité sont de section rectangulaire (d'environ 0,2 x 0,15 à 0,3 x 0,2 mm, dépendant de la largeur des découpes et de l'épaisseur du tube).

De telles parties rigides présentent un taux de recouvrement inférieur à 20 % après expansion.

Ladite partie souple médiane est constituée de fils de diamètre voisin de 0,15 à 0,20 mm. Compte tenu du nombre de lumières complètes des parties rigides d'extrémité et par conséquent du nombre d'ailettes (8), sur lesquelles sont soudés lesdits fils, le maillage est constitué par le croisement de 8 fils en hélice pas à droite avec 8 fils en hélice pas à gauche. Lesdits fils, selon la longueur souhaitée pour ladite partie souple médiane (de 7 à 21 mm), effectuent de 1/4 à 3/4 de tour. L'angle desdits fils, mesuré par rapport à l'axe de l'ensemble, est d'environ 15° avant expansion (préangulation), 45° après expansion.

Une telle partie souple présente un taux de recouvrement voisin de 15 %.

**Revendications**

1. Endoprothèse pour implantation transluminale, caractérisée en ce qu'elle consiste en la juxtaposition alternée de parties cylindriques axialement rigides (1, 1') et d'au moins une partie cylindrique axialement souple (2) ; ladite endoprothèse comportant des parties cylindriques axialement rigides (1, 1') en ses deux extrémités ; lesdites parties cylindriques axialement rigides (1, 1') étant susceptibles de s'expanser radialement de façon plastique et la(les)dite(s) partie(s) cylindrique(s) axialement souple(s) (2) étant susceptible(s) de s'expanser radialement de façon élastique.

2. Endoprothèse selon la revendication 1, caractérisée en ce qu'elle comporte entre deux extrémités cylindriques axialement rigides (1, 1'), susceptibles de s'expanser radialement de façon plastique, une partie cylindrique axialement souple (2) susceptible de s'expanser de façon élastique.

3. Endoprothèse selon la revendication 1, caractérisée en ce que les parties cylindriques axialement rigides intermédiaires et d'extrémité (1, 1') sont identiques.

4. Endoprothèse selon l'une quelconque des revendications 1 à 3, caractérisée en ce que lesdites parties cylindriques axialement rigides (1, 1') et axialement souple(s) (2) sont solidarisées par soudure.

5. Endoprothèse selon l'une quelconque des revendications 1 à 4, caractérisée en ce que les extrémités des parties cylindriques axialement rigides (1, 1') "rebiquent" lors de l'expansion radiale desdites parties.

6. Endoprothèse selon l'une quelconque des revendications 1 à 5, caractérisée en ce que lesdites parties cylindriques axialement rigides (1, 1') consistent en des tubes, selon les génératrices desquels sont distribuées de façon régulière des lumières (3) et des "demi-lumières" (4), lesdites "demi-lumières" (4) fendues (5) débouchant sur les extrémités desdits tubes et générant ainsi des ailettes (6), sur lesquelles sont avantageusement solidarisées lesdites parties cylindriques axialement souples (2).

7. Endoprothèse selon la revendication 6, caractérisée en ce que les dimensions et la distribution desdites lumières (3) et "demi-lumières" (4) sont telles que les parties métalliques pleines desdits tubes présentent toutes sensiblement la même largeur.

8. Endoprothèse selon l'une des revendications 6 ou 7, caractérisée en ce que lesdites lumières (3) et "demi-lumières" (4) ont leurs extrémités arrondies.

9. Endoprothèse selon l'une quelconque des revendications 1 à 8, caractérisée en ce que lesdites parties cylindriques axialement souples (2) consistent en un maillage de fils (7) ; chacun desdits fils (7) étant, en ses deux extrémités, soudé aux parties cylindriques axialement rigides (1, 1') à proximité d'un noeud (8) dudit maillage.

10. Endoprothèse selon la revendication 9, caractérisée en ce que les caractéristiques des mailles dudit maillage - angle au repos, angle ouvert, comportement à l'expansion - sont quasi identiques à celles de la structure tissée du ballonnet d'expansion, utilisé pour la mise en place ce ladite endoprothèse.

11. Endoprothèse selon l'une quelconque des revendications précédentes, caractérisée en ce que :

   - ses parties cylindriques axialement rigides (1, 1') consistent en des tubes selon les génératrices desquels sont distribuées de façon régulière des lumières (3) et des "demi-lumières" (4), lesdites "demi-lumières" (4) fendues (5) débouchant sur les extrémités desdits tubes et générant ainsi des ailettes (6) ;
   - ses parties cylindriques axialement souples (2) consistent en un maillage de fils (7), les fils (7) dudit maillage étant, à proximité d'un noeud (8) de celui-ci, soudés par paire, un à chaque extrémité desdites ailettes (6), un en hélice pas à droite, l'autre en hélice pas à gauche.

## Claims

1. A stent for transluminal implantation, characterized in that it consists in the alternate juxtaposition of axially rigid cylindrical parts (1,1') and at least one axially flexible cylindrical part (2); said stent comprising axially rigid cylindrical parts (1,1') at its two ends; said axially rigid cylindrical parts (1,1') being adapted to expand radially in a plastic manner and said axially flexible cylindrical part (2) or parts being adapted to expand radially in an elastic manner.

2. The stent according to claim 1, characterized in that it includes, between two axially rigid cylindrical ends (1,1') adapted to expand radially in a plastic manner, an axially flexible cylindrical part (2) adapted to expand in an elastic manner.

3. The stent according to claim 1, characterized in that said axially rigid cylindrical intermediate and end parts (1,1') are identical.

4. The stent according to anyone of claims 1 to 3, characterized in that said axially rigid cylindrical parts (1,1') and said axially flexible cylindrical part (2) or parts are welded together.

5. The stent according to anyone of claims 1 to 4, characterized in that the ends of the axially rigid cylindrical parts (1,1') "curl up" during the radial expansion of said parts.

6. The stent according to anyone of claims 1 to 5, characterized in that said axially rigid cylindrical parts (1, 1') consists in tubes, along the generating lines of which are regularly distributed slots (3) and "half-slots" (4), said "half-slots" (4) being split (5) and issuing into the ends of said tubes, thus creating flanges (6) on which the axially flexible cylindrical parts (2) are advantageously fixed.

7. The stent according to claim 6, characterized in that the dimensions and distribution of said slots (3) and "half-slots" (4) are such that the uncut metallic parts of said tubes all have substantially the same width.

8. The stent according to one of claims 6 to 7, characterized in that said slots (3) or "half-slots" (4) have rounded ends.

9. The stent according to anyone of claims 1 to 8, characterized in that said axially flexible cylindrical parts (2) consist in a meshing of wires (7), each one of said wires (7) being welded by its two ends to the axially rigid cylindrical parts (1, 1') close to a knot (8) of said meshing.

10. The stent according to claim 9, characterized in that the characteristics of the meshes of said meshing - natural angle of slope, aperture angle, behavior under expansion - are quasi-identical to those of the woven structure of the expandable balloon used for positionning said stent.

11. The stent according to anyone of the preceding claims, characterized in that :

   - its axially rigid cylindrical parts (1,1') consist in tubes along the generating lines of which are regularly distributed slots (3) and "half-slots" (4) which are split (5) and issue into the ends of said tubes, thereby generating flanges (6);
   - its axially flexible cylindrical parts (2) consist in a meshing of wires (7), the wires (7) of said meshing, close to a knot (8) thereof, welded in pairs, one to each end of said flanges (6), one forming a right-hand helical thread line, and the other a left-hand helical thread line.

## Patentansprüche

1. Endoprothese zur transluminalen Implantation, dadurch gekennzeichnet, daß sie aus der abwechselnden Aneinanderreihung von axial starren zylindrischen Abschnitten (1, 1') und aus wenigstens einem axial nachgiebigem zylindrischen Abschnitt (2) besteht; wobei die Endoprothese an ihren beiden Enden axial starre zylindrische Abschnitte (1, 1') aufweist; wobei die axial starren zylindrischen Abschnitte (1, 1') in der Lage sind, sich radial auf plastische Weise auszudehnen, und der axial nachgiebige Bereich beziehungsweise die axial nachgiebigen Bereiche (2) in der Lage ist beziehungsweise sind, sich auf elastische Weise radial auszudehnen.

2. Endoprothese nach Anspruch 1, dadurch gekennzeichnet, daß sie zwischen zwei axial starren zylindrischen Enden (1, 1'), die in der Lage sind, sich radial auf plastische Weise auszudehnen, einen axial nachgiebigen zylindrischen Abschnitt (2) aufweist, der in der Lage ist, sich auf elastische Weise auszudehnen.

3. Endoprothese nach Anspruch 1, dadurch gekennzeichnet, daß die axial starren zylindrischen Zwischen- und Endabschnitte (1, 1') identisch sind.

4. Endoprothese nach einem der Ansprüche 1 bis 3, dadurch gekennzeichnet, daß die axial starren zylindrischen Abschnitte (1, 1') und axial nachgiebigen zylindrischen Abschnitte (2) durch Schweißen verbunden sind.

5. Endoprothese nach einem der Ansprüche 1 bis 4, dadurch gekennzeichnet, daß die Enden der axial starren zylindrischen Abschnitte (1, 1') bei der radialen Ausdehnung dieser Abschnitte abstehen.

6. Endoprothese nach einem der Ansprüche 1 bis 5, dadurch gekennzeichnet, daß die axial starren zylindrischen Abschnitte (1, 1') aus Rohren bestehen, wobei entlang den Erzeugenden von ihnen auf regelmäßige Weise Öffnungen (3) und „Halböffnungen" (4) verteilt angeordnet sind, wobei die „Halböffnungen" (4), die geschlitzt sind (5), an den Enden der Rohre münden und so kleine Flügel (6) bilden, an denen vorteilhafterweise die axial weichen oder nachgiebigen zylindrischen Abschnitte (2) angebunden sind.

7. Endoprothese nach Anspruch 6, dadurch gekennzeichnet, daß die Abmessungen und die Verteilung der Öffnungen (3) und der „Halböffnungen" (4) derart sind, daß die Vollmetallabschnitte der Rohre alle im wesentlichen dieselbe Breite aufweisen.

8. Endoprothese nach Anspruch 6 oder 7, dadurch gekennzeichnet, daß die Öffnungen (3) und „Halböffnungen" (4) ihre Enden abgerundet haben.

9. Endoprothese nach einem der Ansprüche 1 bis 8, dadurch gekennzeichnet, daß die axial nachgiebigen zylindrischen Abschnitte (2) aus einem Netzwerk oder Maschenwerk von Fäden (7) bestehen; wobei ein jeder dieser Fäden (7) an seinen beiden Enden mit den axial starren zylindrischen Abschnitten (1, 1') in der Nähe eines Knotens (8) des Netzwerks verschweißt ist.

10. Endoprothese nach Anspruch 9, dadurch gekennzeichnet, daß die Eigenschaften der Maschen dieses Maschen- oder Netzwerks - Ruhewinkel, Öffnungswinkel, Verhalten bei Ausdehnung - quasi identisch sind zu jenen der gewebten Struktur der Expansionsgaszelle, die zum Anbringen der Endoprothese verwendet wird.

11. Endoprothese nach einem der vorhergehenden Ansprüche, dadurch gekennzeichnet, daß:

- ihre axial starren zylindrischen Abschnitte (1, 1') aus Rohren bestehen, wobei entlang ihrer Erzeugenden auf regelmäßige Weise Öffnungen (3) und „Halböffnungen" (4) verteilt angeordnet sind, wobei geschlitzte (5) „Halböffnungen" (4) an den Enden der Rohre münden und so kleine Flügel (6) erzeugen;

- ihre axial nachgiebigen zylindrischen Abschnitte (2) aus einem Maschen- oder Netzwerk von Fäden (7) bestehen, wobei die Fäden (7) des Maschenwerks in der Nähe eines Knotens (8) von diesem paarweise verschweißt sind, einer an einem jeden Ende der kleinen Flügel (6), einer in einer rechtsgängigen Schraube, der andere in einer linksgängigen Schraube.

FIG.1

FIG.2

FIG.3

FIG.4